# EUROPEAN PATENT SPECIFICATION

(11) **EP 1 054 959 B1**
(45) Date of publication and mention of the grant of the patent: **25.04.2007**
(21) Application number: 99906425.6
(22) Date of filing: 22.02.1999
(51) Int. Cl.: C12N 15/00, C12N 15/85, C12N 15/86, C12N 5/06, A61K 48/00, A01K 67/027

(54) **HIGH EFFICIENCY METHODS AND COMPOSITIONS FOR INTEGRATING EXOGENOUS DNA INTO GENOMIC DNA OF SPERM**
HOCHEFFIZIENZVERFAHREN UND ZUSAMMENSETZUNGEN ZUM INTEGRIEREN VON EXOGENER DNA IN GENOMISCHE SPERMA-DNA
TECHNIQUES ET COMPOSITIONS PERMETTANT D'INTEGRER AVEC UN TRES BON RENDEMENT UN ADN EXOGENE DANS L'ADN GENOMIQUE DU SPERME

(30) Priority: 22.02.1998 IL 12341198
(43) Date of publication of application: 29.11.2000
(73) Proprietor: Kimron Veterinary Institute, 10021 Bet Dagan (IL)
(72) Inventor: SHEMESH, Mordechai, 43270 Ra'anana (IL); GUREVICH, Michael, 75241 Rishon Le Zion (IL); STRAM, Yehuda, 74084 Nes Ziona (IL); BENVENISTI, Luna, 53488 Givatayim (IL); SHORE, Laurence, S., 76206 Rehovot (IL)
(74) Representative: Ungria Lopez, Javier
(86) International application number: PCT/IL1999/000110
(87) International publication number: WO 1999/042569

(56) References cited:
- WO-A-88/07080
- ZORAQI GRIGOR ET AL: "Integration of foreign DNA sequences into mouse sperm genome" DNA AND CELL BIOLOGY, vol. 16, no. 3, 1997, pages 291-300, XP008037869 ISSN: 1044-5498
- SHEMESH M ET AL: "GENE INTEGRATION INTO BOVINE SPERM GENOME AND ITS EXPRESSION IN TRANSGENIC OFFSPRING" MOLECULAR REPRODUCTION AND DEVELOPMENT, LISSS, NEW YORK, NY, US, vol. 56, no. 2, SUPPL, June 2000 (2000-06), pages 306-308, XP008014700 ISSN: 1040-452X
- AREZZO F., "Sea Urchin Sperm as a Vector of Foreign Genetic Information", CELL BIOLOGY INTERNATIONAL REPORTS, April 1989, Vol. 13, No. 4, pages 391-404, XP002920445
- BRACKETT et al., "Uptake of Heterologous Genome by Mammalian Spermatozoa and its Transfer to Ova Through Fertilization", PROC. NATL. ACAD. SCI. USA, February 1971, Vol. 68, No. 2, pages 353-357, XP002920446
- BRINSTER et al., "No Simple Solution for Making Transgenic Mice", CELL, 20 October 1989, Vol. 59, pages 239-241, XP002920447
- GANDOLFI et al., "The Use of Sperm-Mediated Gene Transfer for the Generation of Transgenic Pigs", J. REPRODUCTION AND FERTILITY, December 1989, Abstract Series No. 4, page 10, XP002920448
- GRUENBAUM et al., "Sperm Cells as Vectors for the Generation of Transgenic Chickens", J. OF CELLULAR BIOCHEMISTRY, 08-26 March 1991, Supplement 15E, p. 194, XP002920449
- KROLL et al., "Transgenic Xenopus Embryos from Sperm Nuclear Transplantations Reveal FGF Signaling Requirements During Gastrulation", DEVELOPMENT, 1996, Vol. 122, pages 3173-3183, XP002920450
- KUSPA et al., "Tagging Developmental Genes in Dictyostelium by Restriction Enzyme-Mediated Integration of Plasmid DNA", PROC. NATL. ACAD, SCI, USA, September 1992, Vol. 89, pages 8803-8807, XP002920451
- LAVITRANO et al., "Sperm Cells as Vectors for Introducing Foreign DNA Into Eggs: Genetic Transformation of Mice", CELL, 02 June 1989, Vol. 57, pages 717-723. XP0002920452
- SCHIESTL et al., "Integration of DNA Fragments by Illegitimate Recombination in Saccharomyces Cerevisiae", PROC. NATL. ACAD. SCI. USA, September 1991, Vol. 88, pages 7585-7589, XP002920453

## Description

### Field of the Invention

The present invention relates to the field of genetic manipulation in biotechnology, more specifically to methods for producing transgenic non-human animals and products derived from said transgenic animals.

### Background of the Invention

Methods for the introduction of a functioning piece of exogenous DNA into the cells of an animal has long been an active area of research and development in molecular biology biotechnology. Such methods, generically known as transgenic technology, allow scientists to study the *in vivo* effects of specific alterations in the genetic complement of an animal, either for the purpose of understanding normal genetic and physiological processes or for dissecting the role of genes in various disease states.

Of equal importance, particularly from an economic perspective, transgenic technology is of great interest as a way to introduce exogenous DNA into animals of commercial importance in order to modify their growth characteristics, to modify the production of desired animal products, or to convert the animals into "factories" for the production of specific proteins or other substances of pharmaceutical interest (Gordon K.E., *et al., Biotechnology* 5:1183-1187 (1987); Wilmut I., *et al., Theriogenology* 33:113-123(1990)).

Early transgenic mice were produced by micro-injection of exogenous DNA into the pronuclei of early embryos (Palmiter, R.D., *et al., Science* 222:809-814 (1983)) or by transfection using viral vectors (Egletis, M.A., *et al., Science* 230:1395-1398. (1985)).

However, these techniques have proved to be of limited use in producing transgenic lines of livestock as their efficiency in larger animals is extremely low, typically on the order of 0.03% to 4.4% (Gandolfi, F., *et al., Reproduction, Fertility, and Development* 8:1050-1060 (1996)). This fact, coupled with the high cost of research with domesticated animals has made the production of transgenic animals of commercial interest extremely expensive. The poor viability of embryos after DNA micro-injection and low integration rate of exogenous genes so introduced, are considered to be mainly responsible for the low success rates in the production of transgenic farm animals (Gagné, M., *et al*., *Theriogenology* 34:417-425 (1990); Wall, R.J., *Journal of Cellular Biochemistry* 49:113-120 (1992)). A review of these methods of producing transgenic animals, and the limitations of these methods, can be found in Palmiter and Brinster, 1986 (Palmiter, R.D. and Brinster, R.L., *Annual Review of Genetics*, 20:465-499 (1986)).

Other methods of producing transgenic animals have been tried with limited success. One such method is nuclear replacement of fertilized ova. Totipotent, *i.e*., immature undifferentiated cells, are transfected *in vitro* by techniques commonly known in the art. The transfected diploid nuclei are isolated by micromanipulation and then transferred into a freshly fertilized egg, after which the "native" male and female haploid pronuclei are removed by suction. The egg cell then proceeds with embryonic development based on the transfected diploid nucleus that was moved into the ovum. However extreme skill is required for the technique of micromanipulation, and the technique itself is quite costly and has a low success rate.

WO 87/05325 discloses a method of transferring organic and / or inorganic material into sperm or egg cells by using liposomes. Liposomes are small, artificial vesicles consisting of a continuous bilayer or a multibilayer of complex lipids. Some of the suspending medium, including any substances contained or dissolved in the suspending medium, are entrapped by liposomes (Smith, A.D., *et al.* editors. *Oxford Dictionary of Biochemistry and Molecular Biology,* Oxford University Press, 1997). The method of WO 87/05325 entails binding liposomes containing DNA (or other substances) to the exterior of sperm and using the liposome coated sperm to fertilize ova *via* artificial insemination. Apparently the liposomes enter the ovum merely as passengers during sperm penetration. However the publication gives no indication that DNA so transferred into the ova actually integrates into the cell's genomic DNA (the *sine qua non* of producing transgenic animals). Furthermore the authors state explicitly that "as the DNA is so tightly packed within the sperms, any attempts at introducing additional genes into the sperms were bound to fail".

Lavitrano, *et al*., (1989) claimed using mouse sperm cells incubated with naked DNA could serve as vectors for introducing exogenous DNA into ova and producing transgenic mice (Lavitrano, M., *et al., Cell,* 57:717-723 (1989)). However they never demonstrated that the DNA was actually transferred into the sperm nucleus or that the exogenous DNA integrated into the sperm cell genomic DNA. Subsequent work by others showed that the exogenous DNA does not enter into the sperm nucleus but simply attaches to, or embeds within, the cell membranes, thus entering the ovum merely as a passenger during fertilization (Bachiller D., *et al., Molecular Reproduction and Development* 30:194-200 (1991); Gandolfi, F., *et al, Reproduction, Fertility and Development* 8:1055-60 (1996)).

More critically, others workers in the field have had difficulty reproducing the results of Lavitrano, *et al*., (1989) either in mice or in other mammalian species, producing very few, if any transgenic animals (Brinster, R.L., *et al., Cell,* 59:239-241 (1989); Hochi, S., *et al., Animal Biotechnology* 1:21-31 (1990); Schellander, K., *et al., Animal Biotechnology* 6:41-50 (1995); Sperandio, S., *et al., Animal Biotechnology* 7:59-77 (1996); Gandolfi, F., *et al, Reproduction, Fertility and Development* 8:1055-60 (1996)). Where transgenic animals were obtained, the exogenous DNA appeared to have been heavily rearranged, thereby negating the very utility of the animal as a transgenic (Sperandio, S., *et al., Animal Biotechnology* 7:59-77 (1996)). Thus the technique of simple incubation of sperm with exogenous DNA appears to be sporadically useful at best.

An alternative method for transferring exogenous DNA into sperm cells is electroporation. This technique creates temporary, short-lived pores in the cell membrane of living cells, by exposing them to a sequence of brief electrical pulses of high field strength. The presence of the these temporary, reversible pores allow cells to take up exogenous material such as DNA, while compromising only slightly the cells' viability (Smith, A.D., *et al*. editors. *Oxford Dictionary of Biochemistry and Molecular Biology,* Oxford University Press, 1997). Electroporation has been used to transfer DNA into eukaryotic cells lines (Knutson J.C. and Yee D., *Annals of Biochemistry*, 164:44-521 (1987); Neumann E., *et al., EMBO Journal* 7:841-845.(1982)) and bacteria (Miller J.F., *et al., Proceedings of the National Academy of Sciences (USA),* 85:856-860 (1988)) resulting in stable transformants.

Gagné and co-workers disclosed the use of electroporation to introduce exogenous DNA into bovine sperm; the sperm was subsequently used to fertilize ova (Gagné, M.B., *et al., Molecular Reproduction and Development* 29:6-15 (1991)). However there was no indication or evidence of integration of the electroporated DNA either in the sperm nucleus or in the nucleus of the egg subsequent to fertilization by the sperm. Indeed the authors state that they could not distinguish whether the electroporated DNA was either tightly bound to the surface of the sperm membrane or embedded within it, but there was no expectation that the exogenous DNA might have been transferred into the nucleus and integrated into the genomic DNA.

Felgner and coworkers disclosed innovative methods and compositions for making liposomes which greatly improved their efficiency for transporting exogenous DNA into cells (Felgner, P.L., *et al., Proceedings of the National Academy of Sciences (USA),* 84:7413-7417 (1987)). Certain cationic lipids or mixtures of certain neutral and cationic lipids have been synthesized which readily form multilamellar liposomes. Upon sonication, multilamellar liposomes will form unilamellar liposomes which will interact spontaneously with DNA to form, with high efficiency, DNA containing liposomes. These can be fused easily with a cell's plasma membrane and thereby be incorporated into the cell's interior (Felgner *et al*., 1987). These cationic liposomes are now commercially available (BRL-Life Technologies), and the technique is known as lipofection (Smith, A.D., *et al.* editors. *Oxford Dictionary of Biochemistry and Molecular Biology,* Oxford University Press, 1997).

Bachiller and co-workers (Bachiller D., *et al., Molecular Reproduction and Development* 30:194-200 (1991)) used such Lipofectin-based liposomes to transfer DNA into mice sperm, and provided evidence that the liposome transfected DNA was overwhelmingly contained within the sperm's nucleus. Despite this result, of 458 offspring produced with the transfected sperm, Bachiller, *et al*. were unable to detect the exogenous DNA in any of the mice, *i.e.*, no transgenic mice could be produced by this technique.

Nakanishi and Iritani (Nakanishi A. and Iritani A., *Molecular Reproduction and Development,* 36:258-261 (1993)) also used Lipofectin-based liposomes to associate exogenous DNA with chicken sperm, which were in turn used to artificially inseminate hens. Although the exogenous DNA was detectable in many of the resultant fertilized eggs, there was no evidence of genomic integration of the exogenous DNA either in the DNA-liposome treated sperm or in the resultant chicks. Worse results were obtained in analogous experiments by Nakanishi and Iritani using electroporation of exogenous DNA into the chicken sperm.

Another method developed for integrating exogenous DNA into yeasts and slime molds is Restriction Enzyme Mediated Integration (REMI). REMI utilizes a linear DNA which is derived from a plasmid DNA by cutting that plasmid with a restriction enzyme that generates single-stranded cohesive ends. The linear, cohesive-ended DNA, together with the restriction enzyme used to produce the cohesive ends, referred to as the corresponding restriction enzyme, is then introduced into the target cells by electroporation. The corresponding restriction enzyme is then thought to cut the genomic DNA at sites that enable the exogenous DNA to integrate *via* its matching cohesive ends (Schiestl, R.H. and Petes, T.D., *Proceedings of the National Academy of Sciences (USA),* 88:7585-7589, (1991); Kuspa A. and Loomis W.F., *Proceedings of the National Academy of Sciences (USA),* 89:8803-8807, (1992)).

Kroll and Amaya (1996) used REMI to insert exogenous DNA into nuclei isolated from *Xenopus laevis* (African clawed toad) sperm. The isolated nuclei were then manually injected into *Xenopus* oocytes to produce transgenic embryos (Kroll, K., and Amaya, E., *Development* 122:3173-3183 (1996)). Although the technique succeeded in yielding many transgenics, the technique required the additional steps of isolating the sperm nuclei from the intact sperm cells, a process which also partially decondenses the sperm nuclear DNA. Furthermore the technique also required that the isolated nuclei be manually injected into the frog oocytes, a laborious procedure requiring an elaborate specialized apparatus, and is practical on a large scales only with the unusually large oocytes characteristic of *Xenopus* (Smith, A.D., *et al.* editors. *Oxford Dictionary of Biochemistry and Molecular Biology,* Oxford University Press, 1997).

Thus it is evident that there is a long felt need for a relatively easy, efficient technique for producing transgenic animals of medical, agricultural and commercial interest, and in numbers large enough to be economically viable. The present invention addresses this need.

### Summary of the Invention

The present invention relates to high efficiency methods and compositions for introducing exogenous double-stranded DNA into non-human sperm cells. More specifically, the methods and compositions of the invention enable the introduced DNA to integrate stably into the genomic DNA contained within the nucleus of the sperm. The method, in part, comprises an adaptation of the Restriction Enzyme Mediated Integration (REMI) technique (Schiestl, R.H. and Petes, T.D., *Proceedings of the National Academy of Sciences (USA)*, 88:7585-7589, (1991), Kuspa A. and Loomis W.F., *Proceedings of the National Academy of Sciences (USA)*, 89:8803-8807, (1992)).

The exogenous DNA to be introduced into the non-human sperm is converted to a linear double stranded DNA possessing single-stranded cohesive ends, by contacting said exogenous DNA with a type II restriction enzyme that upon scission, generates such ends. The DNA to be cut can be a circular DNA such as in a plasmid, such that it possesses at least one recognition and cutting site outside of the genes or regulatory regions critical to the desired, post-integration function of the DNA, and no recognition and cutting sites within the critical regions. Alternatively a linear DNA can serve as the starting material so long as it possesses at least two recognition and cutting sites outside of the genes or regulatory regions critical to the desired, post-integration function of the DNA, and no recognition and cutting sites within the critical regions.

Alternatively the exogenous DNA to be integrated into the non-human sperm nuclear DNA can be prepared by attaching cohesive ends to a linear DNA by various methods well known in the art (Sambrook J., *et al*., (1989): *Molecular Cloning: A Laboratory Manual* 2nd ed., Cold Spring Harbor Laboratory, Cold Spring Harbor, New York). The attached cohesive ends must be identical to the cohesive ends characteristic of a DNA cleaved by a type II restriction endonuclease. The exogenous DNA to be integrated into the sperm genome lacks such cutting sites within the regions critical to the desired, post-integration function of that DNA.

The DNA is then introduced into sperm cells, as is some of a type II restriction enzyme that can cut the sperm genomic DNA at a site or sites that produce cohesive ends identical to those on the exogenous DNA (the corresponding restriction enzyme). The exogenous DNA and the corresponding restriction enzyme can be introduced into the sperm cells, together or sequentially, by electroporation or lipofection or by any other technique that preserves enough of said sperm's motility and fertilization functions, such that the resultant sperm will be to fertilize the appropriate oocytes.

Unexpectedly, this combination of REMI as described in the present application, plus a relatively benign method of transferring exogenous material into a cell (in the examples of the present application electroporation or lipofection), results the exogenous DNA being stably integrated into genomic DNA of a high fraction of the treated sperm, while not diminishing to any great extent, the viability of the sperm or their ability to fertilize non-human oocytes. This outcome is even more surprising in light of the prior art which in word (WO 87/05325) and deed (Kroll, K., and Amaya, E., *Development* 122:3173-3183 (1996)) presumed that stable integration of exogenous DNA into sperm would require at least partial decondensation of the sperm nuclear DNA.

The non-human sperm containing the integrated exogenous DNA can then be used to fertilize eggs by artificial insemination, *in vitro* fertilization, or other fertilization methods known in the art, thereby producing transgenic animals wherein all or a plurality of cells now contain the exogenous DNA stably integrated into their respective genomes.

Also disclosed are transgenic non-human animals or their descendants produced by methods or compositions herein disclosed.

Also disclosed are cells or cell lines derived from transgenic animals or their descendants produced by methods or compositions herein disclosed.

Also disclosed are proteins or other gene products isolated from said transgenic animals or their descendants encoded for by the exogenously introduced DNA.

Also disclosed are compositions of matter produced directly or indirectly by the gene products of the "xogenously introduced DNA.

### Brief Description of the Drawings

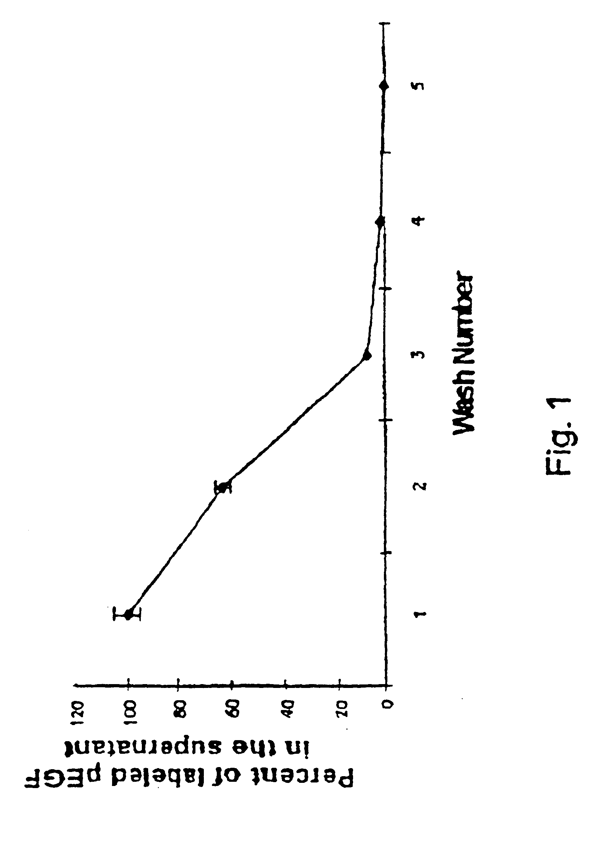

Figure 1 shows captured labeled pEGFP by electroporated sperm cells (10⁷), as determined following five consecutive washings with centrifugation at 700 x G. The reaction was prepared by adding 1 µg/0.4 ml of non-labeled pEGFP and 50,000 cpm of labeled pEGFP, followed by electroporation. Data was expressed as percent of the cpm found in the first washing supernatant.

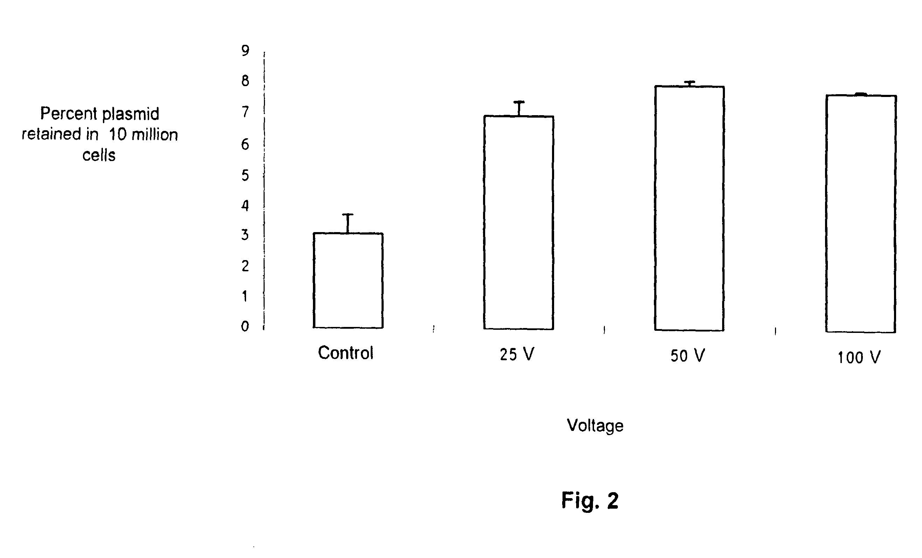

Figure 2 shows the effect of voltage range (0, 25, 50 and 100 V) on percent of radiolabeled plasmids captured by the sperm cells, following electroporation as described in Figure 1.

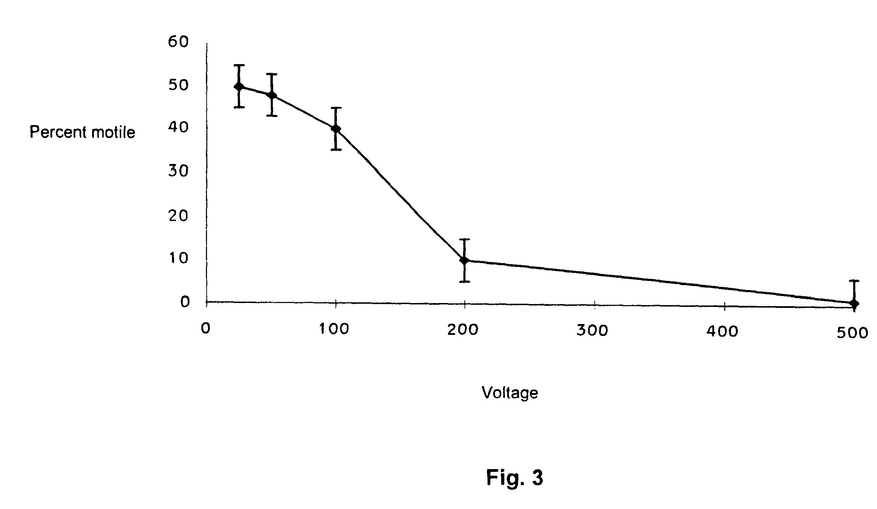

Figure 3 shows the effect of electroporation voltage strength (0, 25, 50 and 100 V) on subsequent sperm motility as determined by video enhanced images. The reaction mixture and electroporation were done as in Figure 1.

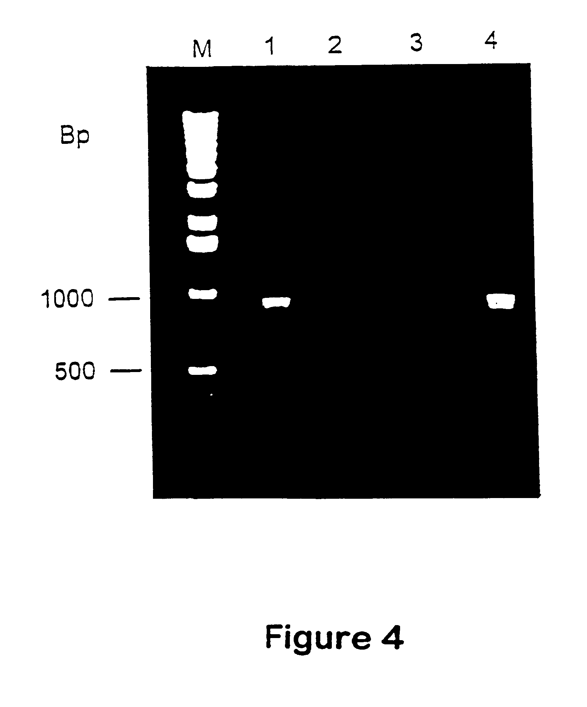

Figure 4 shows the result of PCR of DNA from DNase treated electroporated and non-electroporated sperm cells. Total DNA (1 µg) was amplified as described. Lane 1 represents DNA from pEGFP electroporated and DNase treated sperm. Lane 2 represents DNA from pEGFP not electroporated, DNase treated sperm cells. Lane 3 represents negative control, total DNA from non-treated sperm cells. Lane 4 represents positive control of pEGFP fragment of 911 bp.

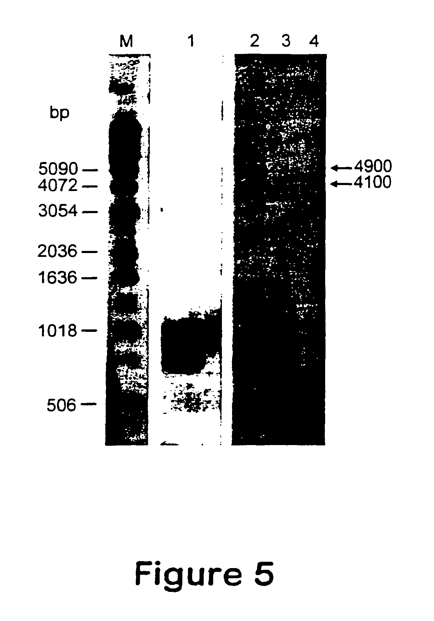

Figure 5 shows the integration of transfected EGFP gene into bovine sperm genomic DNA as detected by Southern blot analysis. Bovine sperm cells were electroporated by *Not*I restriction enzyme and *Not*I linearized pEGFP. Twenty-four hours after electroporation, DNA samples were digested by *HinD*III*,* resolved on 1% agarose and transferred onto Nytran-plus membrane filters. The filter was hybridized with the specific PCR product, 313 bp fragment EGFP gene, which was labeled by chemiluminescent random primer biotin labeling (NEBlot^{™} Phototope^{™} Kit, New England BioLabs). M = Marker DNA (1 Kb ladder, BRL). Lane 1. Free GFP obtained by double digesting of pEGFP with *Not*I and *Hin*DIII. Lane 2. Total DNA extracted from sperm cells electroporated with *Not*I linearized pEGFP and the corresponding restriction enzyme (*Not*I). Lane 3. Total DNA extracted from sperm electroporated without the corresponding restriction enzyme. Lane 4. Total DNA extracted from untreated sperm digested with *Hin*DIII endonuclease.

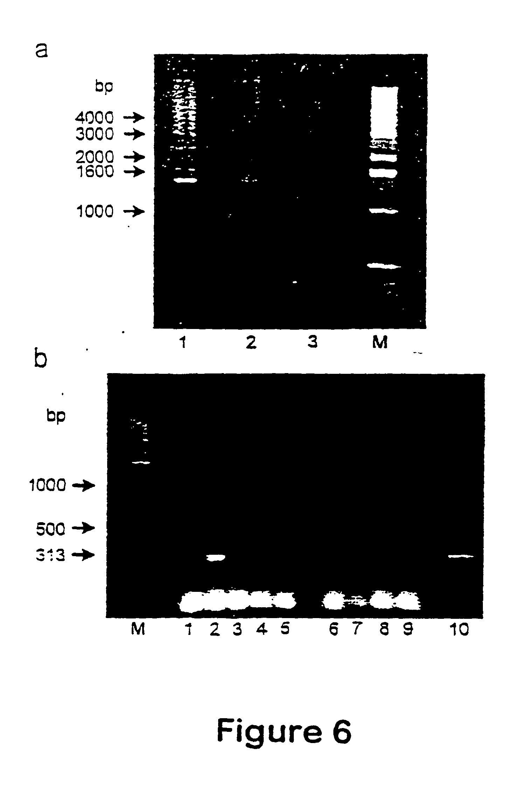

Figure 6 shows the integration of transfected EGFP gene into the bovine sperm genome. Bovine sperm cells were transfected with *Not*I restriction enzyme and *Not*I linearized pEGFP. Twenty-four hours after transfection, DNA was extracted and digested by *Eco*RI. Following separation of the digested DNA by electrophoresis on 1% agarose, designated sites in the digested DNA lanes were needle pricked. The pricked DNA sites were then used as templates in PCR using specific EGFP primers #1 and #2. 6A) Electrophoresis of transfected sperm cell DNA digested by *Eco*RI*.* Lane 1- DNA from sperm cells transfected with the corresponding restriction enzyme; Lane 2 - negative control; digested DNA from non-transfected sperm cells mixed with pEGFP after DNA extraction; Lane 3 - DNA from control transfected sperm cells (incubated with pEGFP in the absence of *Not*I restriction enzyme); Lane M - 1 Kbp ladder as a size marker. 6B) PCR analysis of needle pricked samples recovered from Figure 6a. Lane 1 - DNA sample from Figure 6a at Mr of 1000 bp; Lane 2 - the same as Lane 1 at Mr of 1800 bp; Lane 3 - same as lanes 1 and 2 at Mr of 2000 bp; Lane 4 same as Lanes 1 to 3 at 3000 bp; Lane 5 - same as 1-4 at Mr of 4000 bp. Lanes 6 through 9 are DNA samples from Figure 4a, lane 2 using the same Mr range as those for Lanes 1 to 4. Lane 10 - PCR product of pEGFP as a positive control for the 313 bp fragment. Lane M - 1 Kbp ladder as a size marker.

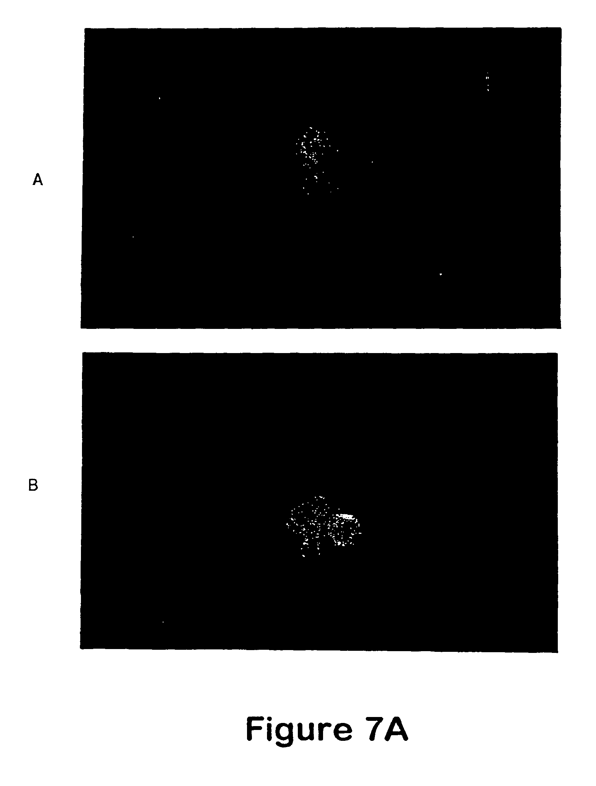

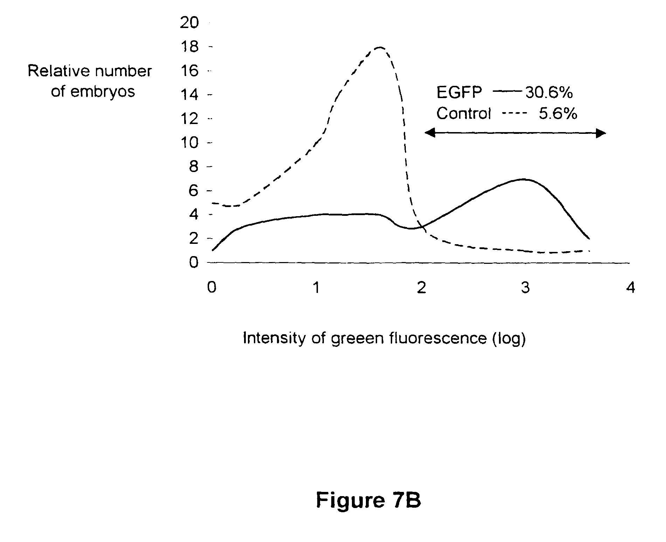

Figure 7 shows EGFP expression in embryos that developed from oocytes fertilized *in vitro* with transfected and non-transfected sperm cells. 7A) EGFP expression in a two cell embryo (top); and EGFP expression in embryonic cells of a bovine morula (bottom). The embryos absorbed blue light and emitted green light as determined by fluorescence microscopy using a standard long pass FITC filter set. 7B) Flow cytometric analysis of EGFP expression in the embryos (72 hours post IVF) as determined by green fluorescence emission after excitation at 488 nm by an argon laser.

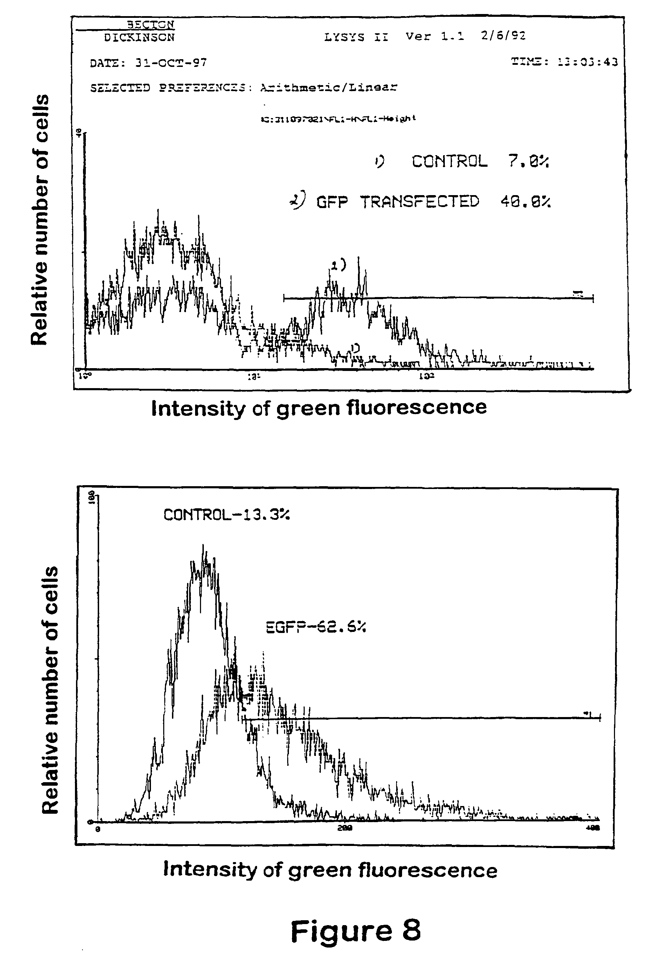

Figure 8 shows the use of flow cytometry to evaluate EGFP expression as determined by green fluorescence emission in avian (top) and bovine (bottom) lymphocytes. Both avian and bovine sperm cells were lipofected with *Not*I linearized pEGFP DNA and *Not*I restriction enzyme. Avian lymphocytes were taken from one month old chicks hatched from hens artificially inseminated with the avian sperm. Bovine lymphocytes were taken from two month old calves born to cows artificially inseminated with the bovine sperm.

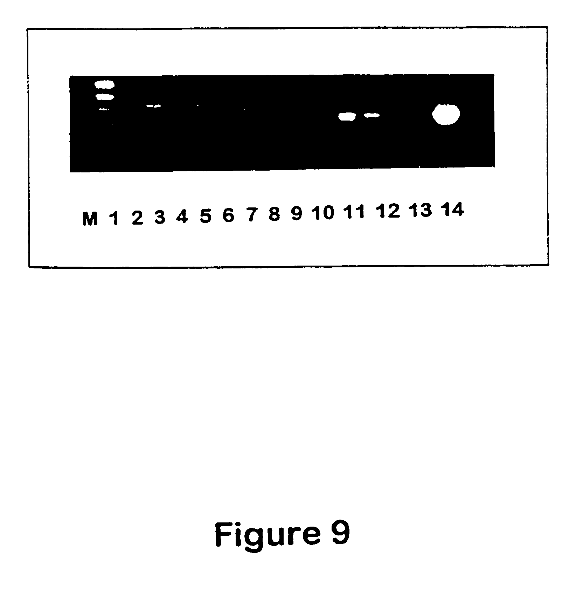

Figure 9 shows the use of PCR products to detect EGFP DNA integration in the chicken lymphocyte. The left most lane (M) is a DNA ladder; Lanes I-II DNA extracted from lymphocytes from chicks produced from *Not*I linearized pEGFP and restriction enzyme *Not*I transfected sperm; Lane 12 DNA from lymphocytes from chicken produced from non-transfected sperm; Lane 13 PCR product in the absence of DNA; Lane 14 positive control. EGFP fragment at 313 bp is evident in lanes 2-6, 8 but not seen in lanes 1 and 7.

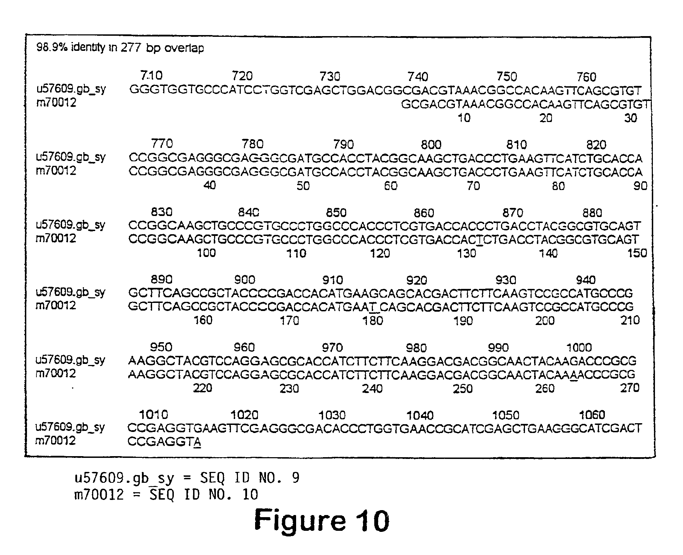

Figure 10 shows the results of automated nucleotide sequencing and homology analysis between *Aequorea victoria* GFP DNA from nucleotide 702-721 and the PCR product were obtained using the primer #1 from Table 1, as described. An homology of 98.9% was found between the *Aequorea victoria* GFP DNA and the PCR product of chick lymphocyte DNA for a 313 bp fragment.

### Detailed Description of Invention

The present invention relates to methods and compositions for stably integrating exogenous DNA into the genomic DNA of non-human sperm. The perm containing the integrated DNA can then be used to fertilize non-human oocytes *via* artificial insemination, *in vitro* fertilization (IVF), or any other method known in the art, to produce a transgenic zygote, embryo, fetus (in mammals), or animal or its descendent(s), or cells or cell-lines derived from any of the foregoing.

Also described are compositions which relate to the sperm with the exogenous DNA stably integrated by the methods of the present invention, and to a transgenic zygote, embryo, fetus (in mammals), or animal or its descendent(s), or cells or cell-lines derived from any of the foregoing, produced from said sperm.

Also described are the non-human transgenic animals or their descendants resulting from the fertilization of non-human oocytes by the non-human sperm containing the integrated exogenous DNA. The transgenic sperm containing the integrated exogenous DNA is used to fertilize oocytes by artificial insemination, *in vitro* fertilization, or other fertilization methods known in the art, thereby producing transgenic animals wherein all or a plurality of cells now contain the exogenous DNA stably integrated into their respective genomes.

The integrated DNA according to the present invention, could contain a gene or genes coding for the production of proteins and / or other gene products, for example a ribozyme, of commercial, industrial, agricultural, and / or medical importance. These products can be isolated from tissues of the transgenic animal or from cells or cell lines derived from the transgenic animal.

In another aspect of the present invention the gene product(s) of the integrated exogenous DNA can be expressed in tissues that produce substances excreted from the animal such as eggs or milk. This allows easy and economical production and purification of the products of the integrated exogenous DNA.

In another aspect, the gene product or products encoded by the exogenous DNA may in turn serve to synthesize a product or products of commercial, industrial, agricultural, and / or medical importance.

In another aspect of the present invention, the exogenous DNA may serve to correct, replace, augment and / or modulate the expression of defective, non-functioning, partially functioning or missing genes. In yet another aspect, the exogenous DNA may serve to modulate, augment and / or enhance the expression of a normally functioning gene or genes. In yet another aspect, the exogenous DNA may serve to confer a new function or capability, for example disease resistance or immunity to an infectious agent. An example of this would be to insert an exogenous DNA that codes for a ribozyme that specifically acts on the nucleic acid genome or the RNA transcripts of an invading virus (Cantor, G., *el al., Proceedings of the National Academy of Sciences (USA)*, 90:10932-10936, (1993), Ojwang, Y., *et al., Proceedings of the National Academy of Sciences (USA),* 92:699-703, (1993), Stram, Y. and Molad, T., *Virus Genes,* 15:33-37 (1997)).

The exogenous DNA of the present invention can be expressed under the control of existing regulatory elements in the cell, especially regulatory elements in the general vicinity of the chromosomal region where the exogenous DNA integrated, and / or regulatory elements present on the exogenous DNA. Examples of such regulatory elements are, but are not limited to, promoters, enhancers, and /or polyadenylation signals.

Examples of promoters contemplated herein include, but are not limited to, promoters from genes such as metalothionein, hemoglobin, actin, muscle creatine, or myosin, or from viruses such as RSV (Rous Sarcoma Virus), ALV (Avian Leukosis Virus), MMTV (Mouse Mammary Tumor Virus), HIV (Human Immunodeficiency Virus), Molony virus, CMV (Cyto-Megalo Virus), EBV (Epstein-Barr Virus), or SV40 (Simian Virus 40). Examples of enhancers include, but are not limited to enhancers from the actin, myosin, hemoglobin, or creatine genes or viral enhancers such as those from RSV, SV40, EBV, or CMV. Examples of polyadenylation signals include, but are not limited to the SV40 polyadenylation signal.

The regulatory elements may or may not limit expression of the exogenous DNA to specific cell and tissue types, specific intra- or extracellular conditions, the presence or absence of particular co-factors, and /or specific temporal factors.

A non-limiting example of an exogenous DNA of the present invention, is a recently developed gene expression reporting system based on the cloned green fluorescent protein gene (GFP) from the jellyfish *Aequorea victoria* (Prasher, D.C, *et al., Gene* 111:229-233 (1992)). The GFP gene and the GFP protein has been used as a fluorescent reporter for gene expression studies in a very wide variety of organisms ranging from bacteria to higher plants and animals (Chalfie M., *et al*., *Science* 263 :802-805 (1994)) including bovine species (Chan A.W.S., *et al., Theriogenology* 47:222 (1997)). The cloning of the GFP gene (Prasher, D.C, *et al., Gene* 111:229-233 (1992); Inoue S. and Tsuji F.I., *FEBS Letters* 341:277-280 (1994)) and its subsequent expression in a wide variety of heterologous systems spanning both the plant and animal kingdoms, has firmly established GFP in the art as a generic and representative reporter system for gene expression.

A commercially available version of the GFP gene, including the necessary regulatory regions, is the plasmid pEGFP-N₃ (Clonetech, Palo Alto, CA). The plasmid DNA encodes an Enhanced Green Fluorescent Protein (EGFP) with a double amino acid substitution (Phe-64 for Leu and Ser-65 for Thy). This protein of 238 amino acids absorbs blue and emits green light after excitation of an intrinsic chromatophore. The EGFP structural gene was expressed under the control of the cytomegalovirus promoter (CMV-GFP).

### REMI

The method of the present invention, of stably integrating exogenous DNA into non-human sperm genomic DNA, comprises in part, an adaptation of the Restriction Enzyme Mediated Integration (REMI) technique (Schiestl, R.H. and Petes, T.D., *Proceedings of the National Academy of Sciences (USA),* 88:7585-7589, (1991), incorporated herein by reference; Kuspa A. and Loomis W.F., *Proceedings of the National Academy of Sciences (USA),* 89:8803-8807, (1992).

The exogenous DNA to be integrated into the sperm nuclear DNA is converted to a linear double stranded DNA possessing single-stranded cohesive ends, by contacting said exogenous DNA with a type II restriction enzyme that upon scission, generates such ends. The DNA to be cut can be a circular DNA such as in a plasmid, such that it possesses at least one recognition and cutting site outside of the genes or regulatory regions critical to the desired, post-integration function of the DNA, and no recognition and cutting sites within the critical regions. Alternatively a linear DNA can serve as the starting material so long as it possesses at least two recognition and cutting sites outside of the genes or regulatory regions critical to the desired, post-integration function of the DNA, and no recognition and cutting sites within the critical regions.

Alternatively the exogenous DNA to be integrated into the sperm nuclear DNA can be prepared by chemically and / or enzymatically adding cohesive ends to a linear DNA by various methods well known in the art (Sambrook J., *el al*., (1989): *Molecular Cloning: A Laboratory Manual*. 2nd ed., Cold Spring Harbor Laboratory, Cold Spring Harbor, New York). The added cohesive ends must be identical to the cohesive ends characteristic of a DNA cleaved by a type II restriction endonuclease. The exogenous DNA to be integrated into the sperm genome must lack such cutting sites within the regions critical to the desired, post-integration function of that DNA. Alternatively the cohesive ends can be added by combining the methods based on type II restriction enzyme cutting and chemical and / or enzymatic addition.

In the present instance and by way of example, the circular pEGFP-N3 plasmid DNA contains a single recognition and cutting site for the type II restriction enzyme *Not*I, this site being outside of any regions critical for expression of the Green Fluorescent Protein gene contained in the plasmid. Upon cutting the circular pEGFP-N3 DNA, *Not*I linearizes the DNA and generates cohesive ends.

According to the present invention, the exogenous DNA, in this instance and by way of example the *Not*I linearized pEGFP-N3 DNA, and the corresponding restriction enzyme in this instance and by way of example *Not*I, can be introduced into the sperm cells together or sequentially, in this instance and by way of example by electroporation, or lipofection.

Preferably electroporation is used to transfer the REMI mix into the sperm, and most preferably lipofection is used. However the present invention contemplates that any technique capable of transferring exogenous material into sperm could be used so long as the technique preserves enough of said sperm's motility and fertilization functions, such that the resultant sperm will be to fertilize the appropriate oocytes.

Unexpectedly, this combination of REMI as described in the present application, plus a relatively benign method of transferring exogenous material int® a cell (in the examples of the present application electroporation or lipofection, but not limited to these methods), results in the exogenous DNA being stably integrated into genomic DNA of a high fraction of the treated sperm, while not diminishing to any great extent, the viability of the sperm or their ability to fertilize oocytes. This outcome is even more surprising in light of the prior art which in word (WO 87/05325) and deed (Kroll, K., and Amaya, E., *Development* 122:3173-3183 (1996)) presumed that stable integration of exogenous DNA into sperm would require at least partial decondensation of the sperm nuclear DNA.

These and other techniques that are used in the examples illustrating the present invention are described below. In addition, other methods well known in the art can be found in Sambrook J., *et al*., (1989): *Molecular Cloning: A Laboratory Manual*. 2nd ed., Cold Spring Harbor Laboratory, Cold Spring Harbor, New York. Definitions of technical words and phrases well known in the art, used herein can be found in Smith, A.D., *et al*. editors. *Oxford Dictionary of Biochemistry and Molecular Biology,* Oxford University Press, 1997.

### Electroporation of Sperm Cells

Frozen chicken sperm was obtained from the Centre for Artificial Insemination (Hasherut, Artificial Insemination Center, Mobile Post Office, Shikmim, Israel). The straws containing the frozen sperm were thawed in a water bath (37°C, 1 minute), washed twice with TALP media (Tyrode's Albumin-Lactate-Pyruvate media; Parrish J., *et al., Theriogenolgy*, 25:591-600 (1986)) and used immediately. 10⁷ sperm cells were added to 1 ml of mannitol buffer (0.3 mM mannitol, 0.4 mM MgCl₂, 0.05 mM CaCl₂, Sigma Chemical Co., St. Louis, MO; Zimmerman U. and Vienkin J., *Membrane Biology* 67:165-182 (1982)).

Green fluorescent protein gene plasmid DNA (pEGFP) was linearized for transgenesis by incubating overnight at 37°C with *Not*I (Promega, Madison, WI). Linearized plasmid were purified for transgenesis using the GeneClean II Kit (BioLabs, Beverly, MA) and eluted in dH₂O at a concentration of 1000 ng/µl.

The reaction mixture of the present invention was prepared for electroporation by adding pEGFP (1 µg/0.4 ml) in a BTX Electroporation Cuvette plus (part no. 620) with a distance of 2 mM between electrodes. For studies requiring radioactively labeled DNA (*e*.*g*., for measuring the amount of exogenous DNA retained by the sperm), 1 µg/0.4 ml unlabelled pEGFP DNA and 1 pgm labeled pEGFP (50,000 cpm/pgm) DNA, labeled with [α³²P]-dCTP by nick translation (Rigby P.W.J., *et al*., (1977) *Journal of Molecular Biology,* 133:237-251 (1977)).

The corresponding restriction enzyme, in this instance and by way of example, *Not*I (100 units/0.4 ml) was added to the electroporation cuvette. The voltage range was from 0 to 1.0 kV with a pulse duration of 40 msec (Electro Manipulator, 2001, BTX, San Diego, CA). The effect of electroporation was tested at 0, 25, 50 or 100 V. The effect of each voltage used was evaluated using the percent of labeled pEGFP insertion into the sperm as well as the effect on motility as determined before and after electroporation.

### Liposomal Insertion of Foreign DNA into Sperm

According to the present invention, exogenous DNA and corresponding restriction enzyme can be transfected into the sperm cells by lipofection. The reaction mixture of the present invention was prepared for lipofection as follows: Liposomes were prepared as described by Felgner and coworkers (Felgner, P.L., *et al., Proceedings of the National Academy of Sciences (USA),* 84:7413-7417 (1987)) using LipofectAMINE^{™} from BRL-Life technologies, using the manufacturers instructions. Liposomes (10 µg/200 µl) were incubated with 1 µg *Not*I linearized pEGFP or 50 Units of *Not*I restriction enzyme for 1 hour. A suspension of the liposomes containing the *Not*I linearized pEGFP and the *Not*I restriction enzyme was then incubated with 10⁷ sperm cells for 1 hour.

### DNase incubation to remove plasmid from sperm cell surface.

To evaluate the fraction of exogenous pEGFP captured in the control and electroporated bovine sperm cells, DNase was used to digest the pEGFP DNA bound to the sperms' extracellular membrane. The sperm into which DNA was electroporated or the control (incubated but not electroporated) sperm were suspended in mannitol buffer, washed once in TALP medium and once in 5 ml of DNase I (RQ1 RNase-free DNase, Promega) buffer [50 mM Tris-HCl, pH 7.5, 10 mM MgCl2, 50 µg/ml fatty acid free bovine serum albumin (BSA FAF, Sigma; Ausubel F.M., *et al., Current Protocols in Molecular Biology* (1987) New York. Greene Publishing, Wiley Interscience pp 3.13.1-3.13.2.), centrifuged at 750 g for 10 minute at 20°C. Supernatant was removed and the pellet resuspended in 1 ml of the same buffer. Four units of DNase I were added to 1 ml resuspended sperm cells and incubated for 15 minute at 37°C. The reaction was arrested by the addition of 25 µl of 0.5 M EDTA. DNase treated, electroporated sperm and control (incubated but not electroporated) sperm were then washed four times in 5 ml TALP medium and used for total DNA extraction. PCR was used to amplify the plasmid DNA using the pair of primers shown in Table 1 for the 911 bp fragment.

### In Vitro Maturation and Fertilization of Oocytes

Ovaries from cycling cows were removed within 30 minute after slaughter and used as a source of ova as previously described (Gurevich, M., *et al., Israel Journal of Veterinary Medicine* 48:84-87 (1993)). Briefly, the cumulus oocyte complexes (COC) were aspirated from 1 to 5 mM follicles with 18 g needle, pooled and selected in accordance to cumulus appearance. The COC were washed three times in HEPES buffered Tyrode's medium (TLH) (Bavister R.D., *el al., Biology of Reproduction* 28:235-247 (1983)), supplemented with 0.2 mM pyruvic acid (Sigma), 0.9 mg/ml glucose, 10% fetal calf serum (heat treated FCS, GIBCO Lab, Grand Island, NY), and 0.05 mg/ml gentamycin (Sigma). Oocyte maturation was carried out as previously described (Gurevich, M., *et al., Israel Journal of Veterinary Medicine* 48:84-87 (1993)).

*In Vitro* fertilization (IVF) was performed using fertilization medium (Modified TLH, 10.6% BSA-FAF 0.2 mM pyruvic acid, 10 µg/ml heparin (Sigma) and 0.05 mg/ml gentamycin (Bavister, B.D and Yanagimachi , R., *Biology of Reproduction* 16:228-237(1977)) in droplets (45 µl) containing 5 COC. The fertilization media droplet were placed in sterile Petri dished under mineral oil and equilibrated for 2 h in culture conditions prior to use. Five microliters of electroporated sperm cell suspension (5.0x104 cells) were directly added into each droplet.

### Development of Morulas

*In vitro* development of embryos was obtained with a co-culture of bovine granulosa cells as previously described (Gurevich, M., *et al., Israel Journal of Veterinary Medicine* 48:84-87 (1993)). Culture medium was replenished every 48 h by adding 50 µl of fresh TCH-199 FCS. After 5 days, the resulting compacted morula were evaluated for development.

### Detection of Fluorescence

EGFP (Enhanced Green Fluorescent Protein, the product of the pEGFP DNA) fluorescence was determined by light microscopy using Nikon fluorescence microscope (Nikon Diphot 300 stereo-microscope with Nomarski optics) with a standard fluorescein isothiocyanate (FITC) filter set (excitation BP450-490 nm, dichroic 510 nm, emission LP 520 nm). Alternatively, cells emitting green fluorescence were analyzed by flow cytometry (Becton Dickinson Immunochemistry Systems, San Jose CA) by excitation with an argon laser emitting at 488 nm. The FL1 emission channel (normally used to detect FITC) was used to monitor EGFP fluorescence.

### Sperm DNA Extraction

Sperm nuclear DNA was extracted using 1% SDS, 50 mM DDT and proteinase K (0.4 mg/ml) incubated at 60°C overnight as described (Ron M., *et al., Animal Genetics* 25:259-264 (1994)).

### Southern Blot Analysis

Genomic DNA was extracted from *Not*I linearized pEGFP with or without *Not*I restriction enzyme transfected sperm cells and then digested with *Hin*DIII by incubating at 37°C overnight and electrophorating on a 1% agarose gel in 1 X Tris-acetate buffer (Sambrook J., *et al*., (1989): *Molecular Cloning: A Laboratory Manual.* 2nd ed., Cold Spring Harbor Laboratory, Cold Spring Harbor, New York) at 100 V for 1 h. Samples were then denatured with 0.5 M NaOH and blotted onto Nytran-plus, membrane filters (Schleicher & Schuell, Dassel, FRG) using trans-vacuum blotter TE 80 (Hoefer, Uppsala, Sweden) in 25 mM Na₂PO₄, pH 6.5 for 1 h. The membranes were subsequently irradiated with short wave UV for 2 minutes at 1200 µWatts cm⁻² as described by Sambrook *et al*. (1989). The filter was hybridized overnight at 65°C with the specific PCR product, 313 bp fragment of EGFP gene, which was labeled by chemiluminescent random primer biotin labeling (NEBlot^{™} Phototope^{™} Kit, New England BioLabs). Pre-hybridization for 1 h at 65°C in 10-20 mL hybridization buffer was also performed.

### Cloning of the IBDV Large ORF Gen

The details of the cloning of the Infectious Bursal Disease Virus (IBDV) Large ORF gene, to produce plasmid pKVI112, are described elsewhere in preliminary US patent application 0231.00.10. A summary of the key features of the cloning procedure is disclosed below:

RNA Extraction: RNA was extracted from IBDV infected chicken bursal tissue using the acid phenol, guanidine thiocyanate technique (Chomczynski, P. and Sacchi, N., *Anal. Biochem.* 162:156-159 (1987)), using Tri-Reagent LS (Molecular Research Center, Cincinnati, OH) according to manufacturer's instructions.

RT-PCR: The RT reaction was perform in 500 µl thin wall tube and contained 2 µl 100 ng/µl RNA template in water, 2 µl of the reverse primer, GTA**GCGGCCGC**CTTACTCAAGGTCCTCGTCAGAGAC (SEQ ID NO. 1) at position 3044-3068 (100ng/µl), 5.9 µl RNase free water and mineral oil was added then incubated at 70°C for 10 min., 92°C for 2 min. and put immediately at 4°C. Then we added 4.0 µl 5x RT reaction mix (Promega, Madison, WI), 0.5 µl RNasine 40 u/µl (Promega), 0.4 µl mix of 2.5 mM each dNTP and 0.2 µl 1M DTT. The tube was incubated at 42°C for 2 min. and then after 30 min. incubation at 4°C 5 µl of 15 U of AMV RT in dilution buffer (10% glycerol, 10 mM K phosphate pH 7.4, 0.2% Triton X-100 and 2 mM DTT) was added. Incubation proceeded for at least 70°C at 42°C then put immediately at 4°C. To the PCR mix (10 mM Tris-HCl pH 8.3, 50 mM KCl, 1.5 mM MgCl₂, 0.001 w/v gelatin, 5 µl dNTP (2.5 mM each), 1 µl of each of the two primers, 2.5 U of Ex-Taq (Takara, Kyoto, Japan), 2 µl from the RT reaction and water were added to bring the reaction mix to final volume of 50 µl).

Reaction conditions were 95°C for 2 min, then 14 cycles of 55°C for 1 min., 68°C for 3 min., 98°C for 20 sec. then 16 cycles of 55°C for 1 min., 68°C 3 min. + 15 sec/cycle, 98°C for 20 sec. then a final cycle of 55°C for 1 min. and 72°C for 10 min. Two viral fragments were amplified to clone the entire IBDV ORF. A fragment from base 15 to 1830 using primer GTA**GCGGCCGC**AAACGATCGCACATATGACAAACCT (SEQ ID NO. 2) carrying a *Not*I site as a forward primer and GTATGAA**GGATCC**TCTTTTGAGATG (SEQ ID NO. 3) carrying the unique viral *BamH*I site as the reverse primer. The second fragment from base 1807 to 3068 using ATCTCAAAGA**GGATCC**TTCATACGA (SEQ ID NO. 4) from base 1807 to 1831 and carrying the viral BamH I site and the reverse primer GTA**GCGGCCGC**CTTACTCAAGGTCCTCGT CAGAGAC (SEQ ID NO. 5) carrying a *Not*I site. The two amplified fragment were cloned into pGEM-T Easy plasmid (Promega) using the TA cloning system (Newton, C. R. and Graham, A., (1994). In: *PCR,* BIOS Scientific Publishing, Ltd., Oxford, United Kingdom).

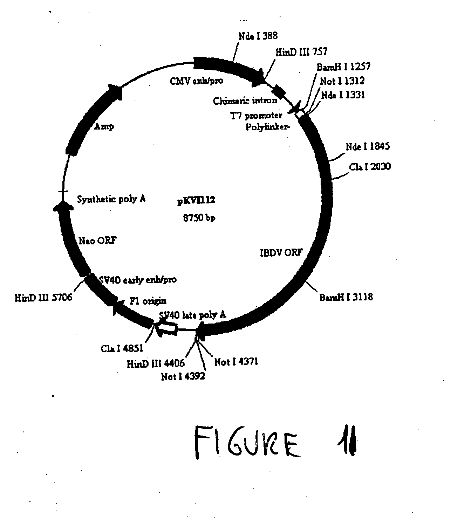

To purify the two fragments, the resultant plasmids were double digested with *BamH*I and *Not*I and separated from the vectors by electrophoresis on a 1% agarose gel (Sambrook J., *et al*., (1989): *Molecular Cloning: A Laboratory Manual*. 2nd ed., Cold Spring Harbor Laboratory, Cold Spring Harbor, New York). The two fragments were purified from the gel and cloned into the pTargeT expression vector (Promega) to create the pKVI112 plasmid illustrated in figure 11.

### Examples

The following Examples include representative examples of aspects of the present invention. The Examples are intended to illustrate, exemplify, and highlight various aspects of the present invention and not to limit its scope in any way or manner.

### Example I

The present invention provides for the stable integration of exogenous DNA into viable spermatozoa *via* electroporation, while preserving their viability and utility.

Electroporation conditions for optimizing viability together with DNA retention determined as follows.

To determine the percentage of pEGFP DNA retained by electroporated spermatozoa, 10⁷ sperm Bovine cells were incubated in the presence of ³²P radiolabeled (1 pgm) and non-labeled (1 µg/0.4 ml) linearized pEGFP. The incubation mixture was electroporated over the voltage range from 0 to 100 V. After five successive washings of the electroporated bovine sperm cells, free plasmid DNA could not be detected in the wash supernatant as determined by the level of radioactivity in the supernatant (Fig 1). However, the radioactivity was found in the sperm cell sediment after the washings, indicating that the plasmid DNA was strongly captured by the electroporated sperm.

The effect of electroporation voltage on DNA capture by the electroporated sperm cell and the effect on motility are shown in Fig. 2 and Fig 3. 50 V for 40 msec was the optimal voltage for DNA capture with the least adverse effect on motility over the range tested. This dosage was therefore used for the subsequent experiments for gene insertion. The level of captured labeled plasmid pEGFP DNA in the electroporated sperm cells were significantly higher than the non-electroporated controls (P<0.01).

The amount of pEGFP DNA captured in the sperm cells after electroporation as compared to the non-electroporated controls, was also evaluated. Both electroporated and non-electroporated sperm were exposed to DNase I as described above and PCR was used to amplify the plasmid DNA using primers #2 and #3 as shown in Table 1. The 911 bp fragment of pEGFP was detected only in electroporated sperm cell DNA after DNase treatment, but was not detected in the non-electroporated control (Fig. 4). The lack of PCR a product synthesized from the DNA isolated from the non-electroporated sperm following DNase treatment, indicates that pEGFP DNA bound to the surface membrane of the controls where it was destroyed by the DNase and hence the DNA was not transferred into the cell's interior. By contrast, the PCR product detected in the electroporated sperm even after the DNase treatment indicates that the pEGFP DNA was internalized by the sperm as a result of electroporation.

**Table 1. Sequences of EGFP gene and plasmid-EGFP oligonucleotide primers used for PCR.**

| Gene | Primer sequences | Nucleotide | Product Size (Bp) |
|---|---|---|---|
| EGFP | 5' primer (SEQ ID NO. 6) | | |
| #1 | 5'-ACCGGGGTGGTGCCCATCCT-3' | 702-721 | 313 |
| | 3' primer (SEQ ID NO. 7) | | |
| #2 | 5'-TTCACCTCGGCGGGGTCTT-3' | 996-1015 | |
| pEGFP | 5' primer (SEQ ID NO. 8) | | |
| #3 | 5'-AACGACCCCCGCCCCATTGAC-3' | 104-123 | 911 |
| | 3' primer (SEQ ID NO. 7) | | |
| #2 | 5'-TTCACCTCGGCGGGGTCTT-3' | 996-1015 | |

### Example 2

Electroporation mediated REMI of bovine sperm cells was performed as described above. Briefly, pEGFP DNA was linearized with cohesive ends by incubation overnight with *Not*I restriction endonuclease. The *Not*I linearized pEGFP DNA and *Not*I restriction endonuclease were electroporated into bovine sperm as described.

To demonstrate that the captured pEGFP in the electroporated sperm is integrated into the sperm genome after electroporation mediated REMI, total DNA was extracted and Southern blot analysis was performed, using *Hin*DIII digested DNA and the 313 PCR fragment as a probe. As can be seen in Fig. 5, two fragments were detected by the probe, one with a Mr of 4.1 Kbp and the other with an Mr of 4.9 Kbp, *i*.*e*., in a fragment size larger than the 4.7 Kpb length of the plasmid. These fragments were detected by the probe exclusively in the DNA extract of the sperm cells electroporated with the *Not*I treated pEGFP DNA and *Not*I enzyme, *i*.*e*., the corresponding restriction endonuclease. pEGFP DNA was detected at Mr 4.7 Kbp and free EGFP at 768 bp in partially *Eco*RI digested pEGFP. These results strongly suggest that the pEGFP DNA was integrated into a unique site in the sperm genome, in most if not all of the sperm cells electroporated with the *Not*I treated pEGFP DNA and *Not*I enzyme.

### Example 3

To compliment the findings in the Southern blot, a second technique using PCR of needle pricked DNA was used. Bovine sperm cells were electroporated with *Not*I and *Not*I linearized pEGFP as described. Twenty four hours after electroporation, the DNA was extracted and digested by *Eco*RI. Following separation of the digested DNA by electrophoresis on 1% agarose, designated sites in the digested DNA lanes (1000-4000 bp) were needle pricked. The pricked DNA sites were then used in PCR for each site using specific EGFP primers #1 and #2.

The EGFP sequence was found exclusively in digested DNA isolated from the sperm cells electroporated with *Not*I linearized pEGFP DNA and *Not*I restriction endonuclease, mainly at Mr of 1800 bp, but a weak signal was also seen at 3000 bp (Fig. 6a and 6b). The absence of EGFP in lower (1000 bp) and higher(4000 bp) molecular weight DNA) of the electroporation mediated REMI sperm cell DNA (Fig. 6b) as well as in the mixed DNA control, suggests that the presence of EGFP DNA in the transfected sperm cells is authentic, *i*.*e*., is integrated into the genomic DNA, and is not due to DNA trapping. The lack of the 313 bp fragment in the absence of *Not*I suggest that the electroporation of the restriction enzyme is essential for gene integration.

### Example 4

Transgenic bovine sperm prepared by electroporation mediated REMI was used for *in vitro* fertilization (IVF) of bovine oocytes as described earlier. The use of electroporated sperm cells in IVF resulted in a decrease in the number of cleaved oocytes (69% vs 42%). However EGFP was fully expressed in all the blastomeres in 5-18% of the morula (Table 2) as determined with a fluorescence microscope equipped with FITC filter set.

**Table 2. Expression of EGFP by pEGFP DNA in bovine morula resulting from IVF of matured oocytes fertilized by bovine sperm electroporated with NotI linearized pEGFP DNA and NotI enzyme (+) and untreated bovine sperm cells (-). Data expressed as percent of full expression of EGFP in all blastomeres of the morula as determined by determined by fluorescence microscopy, using a standard FITC filter set. Morula were excited with 450-490 nm light and the subsequent emission of green fluorescence above 520 nm was noted.**

| Exp. #. | Transfection | Cleavage | Expression of EGFP in Morula |
|---|---|---|---|
| 1 | - | 64% (58/91) | - |
| | + | 37% (20/54) | 15% (3/20) |
| 2 | - | 73% (61/84) | - |
| | + | 49% (19/39) | 5% (1/19) |
| 3 | - | 70% (49/70) | - |
| | + | 43% (22/51) | 9% (2/22) |
| 4 | - | 62% (61/97) | - |
| | + | 37% (11/30) | 18% (2/11) |

EGFP was also partially expressed in a mosaic pattern in some morula. EGFP expression in two cell embryos and morula as determined qualitatively by the emission of green light *via* fluorescence microscopy (Fig. 7A) and determined quantitatively *via* flow cytometry (Fig. 7B).

### Example 5

Exogenous DNA was also integrated into chicken sperm, and live transgenic animals produced by the methods of the present invention. Liposome mediated transfection, known in the art as lipofection, was used instead of electroporation to enable the REMI into live chicken spermatozoa.

Liposomes preparations containing *Not*I treated pEGFP DNA, and *Not*I restriction endonuclease were prepared as described above. Chicken sperm was lipofected with the liposome suspensions described above, and the sperm was subsequently used for artificial insemination of cycling hens. Fertilized eggs were incubated and hatched. The percent of hatched eggs was not different from that of controls inseminated with non-lipofected sperm.

At three weeks of age, blood samples were taken from the hatched chicks and the white blood cells were separated for analysis. Flow cytometric analysis indicated that 17/19 of the chicks expressed EGFP (Fig. 8 top). 30-50% of the white blood cells exhibited the green fluorescent light characteristic of EGFP. The total DNA from the white blood cells, contained the 313 bp EGFP fragment as shown by PCR (Fig. 9).

Nucleotide sequencing of the chicken white blood cell DNA indicated a sequence which was 98.9% homologous to that of EGFP (Fig. 10).

A second generation of chicks was produced by mating transgenic sires to non-transgenic hens. Lymphocytes taken from the chicks were examined for the presence and expression of the EGFP gene. The EGFP gene was found and expressed in 83.3% (25/30) of the second generation chicks, using the same techniques of FACS, PCR and DNA sequence analysis as described earlier.

Blood samples were also taken from transgenic two month old calves born to cows artificially inseminated with transgenic bovine sperm lipofected with pEGFP DNA using the method of the present invention, and the white blood cells were seperated for analysis. Flow cytometric analysis indicated that about 60% of the white blood cells expressed EGFP (Fig. 8 bottom).

### Example 6

The method of the present invention can be used to produce animals resistant to various diseases, by transfecting sperm with an exogenous DNA that encodes a factor, such as an antibody, that directly neutralizes the pathogen. Alternatively, the exogenous DNA could encode a factor that interferes with the pathogen's normal replication mechanism, thereby minimizing or eliminating the infecting pathogen's ability to spread within the body of the animal.

By way of example, birds were produced resistant to the Infectious Bursal Disease (IBD) virus using the method of the present invention. IBDV is a member of the birnavirus family, characterized as non-enveloped icosohedral viruses with an RNA genome comprised of two double-stranded RNA molecules (Dobos, P., *et al., Journal of Virology* 32:593-605 (1979)). IBDV infections occur worldwide in all major poultry producing areas. The incidence of infection in these areas is high; essentially all flocks are exposed to the virus during the early stages of life. Highly virulent strains originally isolated in the Netherlands in 1989 are the cause of severe outbreaks with high mortality. No therapeutic or supportive treatment is available; conventional immunization of chickens is the principle method used to control the disease in chickens (Lukert, P. D. and Saif, Y. M., "Infectious Bursal Disease", p. 721-735 in, *Diseases of Poultry, 10^{th} Ed*., Calnek, B. W., *et al*., editors. Mosby Wofe, 1997).

The IBDV ORF A1 sequence encodes three genes; VP2 and VP3 are the two viral structural proteins which form the viral capsid. The third ORF A1 gene, VP4, is a protease responsible for processing the large polyprotein into the individual capsid proteins (Hudson, P. J., *et al., Nucleic Acids Research,* 14:5001-5012 (1986)). Expression of a recombinant IBDV ORF Al sequence in cultured cells results in the accumulation of paracrystalline arrays of IBDV virus-like particles, that are antigenicly and structurally akin to authentic IBD virus particles (Fernández-Arias, A., *et al., Journal of General Virology,* 79:1047-1054 (1998)).

Although the intracellular expression of certain recombinant viral genes can negatively regulate viral replication in some plant viral systems (*e.g.*, Potato Virus Y in potato plants - Vardi, E., *et al., Proceedings of the National Academy of Sciences (USA),* 90:7513-7517 (1993); Livneh, *et al., Transgenics,* 1:565-571 (1995)), there are no teachings or suggestions in the prior art that an animal could be made resistant to a viral disease by integrating an exogenous viral gene into the genome of the animal's cells. Even if a candidate viral gene was successfully identified and integrated into an animal's genome, constitutive expression of a foreign gene could easily interfere with the cell's normal function and hence viability, either as individual cells in culture or even more so as a whole animal. Thus it was unexpected that the method of the present invention was used successfully to produce transgenic animals resistant to an infectious disease. In the particular example described herein, IBDV ORF Al transgenic, live chickens were produced that proved resistant to Infectious Bursal Disease.

The IBDV ORF Al sequence was isolated and cloned into an expression plasmid to create pKVI112, as described in a separate US provisional patent application 0231.00.10 and was summarized earlier. Plasmid pKVI112 was transfected into rooster sperm by REMI transfer using lipofection and the restriction enzyme *Not* I. The resultant sperm was used to artificially inseminate eight birds. A control group of eight birds were inseminated with sperm obtained from the same source, but transfected with a plasmid which did not contain the IBDV ORF A1 sequence.

Thirty eggs from each group were collected, incubated and hatched (97%). At two month of age, the chicks were intraorally and intraocularly inoculated with a high titer (10³ EID₅₀ (mean Embryonic Infective Dose 50)) of IBD virus. None of the birds in either the control or IBDV ORF A1 sequence plasmid group displayed clinical signs of IBD after the usual incubation time of three weeks post-inoculation. Immunological examination of the sera showed that the birds in both group were producing antibodies to IBDV before the challenge with IBDV inoculation, presumably as a result of some serendipitous exposure to the virus.

Despite the lack of overt external IBD symptoms, the chicks were killed and bursal tissue was taken from both the IBDV plasmid transfected for histological examination, immediately following the usual incubation time of three weeks post-inoculation. Results from the experimental and control groups were obtained by comparing the weights of the bursae, as a percentage of the body weight of the chick from which the bursa was taken, and by a histological examination of the bursae. Unexpectedly, the bursal weight as a percentage of body weight on average, indicated that the bursal weight of the group transfected with the IBDV ORF A1 sequence containing plasmid was about twice that of the controls (1.82±0.06 bursa to body weight ratio in the IBDV ORF A1 sequence transfected birds *versus* a ratio of 0.99±0.05 in the control birds; N=12 (6 birds in each group) P<0.01)

Subsequent histological examination of the control group bursae revealed clear, characteristic signs of IBD, notably extrusion of lymphocytes from the follicles, loss of bursa follicle structural integrity and necrosis of the tissue. By contrast, the follicles in the bursae of all the birds in the IBDV ORF A1 sequence transgenic group had a normal structure. There were no significant signs of IBD.

Thus it is evident the expression of the IBDV ORF A1 sequence in the cells of the chicks produced from the sperm made transgenic by the method of the present invention, conferred significant resistance to the IBDV virus, over that of the control group. The presence of circulating anti-IBDV antibody in the control group may have delayed somewhat the onset of overt external IBD symptoms but it clearly did not prevent the disease.

While not bound by theory, it is believed that the IBDV ORF A1 sequence in the cells of the experimental chickens, directs the synthesis of sufficient quantities of the three recombinant viral proteins (VP2, VP3, and VP4) to prevent the replicate synthesis of the inoculated challenge IBD virus. As a result the infecting pathogen is unable to produce the progeny viral particles needed to induce the clinical symptoms and manifestation of the disease in the bursa of the IBDV ORF Al transgenic chickens.

## Claims

1. A method of integrating exogenous double stranded DNA into genomic DNA of non-human sperm comprising,
a. forming cohesive ends on the exogenous DNA, such that said cohesive ends are identical to the cohesive ends characteristic of a DNA cleaved by a given type II restriction endonuclease; and
b. transferring said exogenous DNA and said type II restriction endonuclease into viable sperm.

2. The method of Claim 1 wherein the exogenous DNA and type II restriction endonuclease are transferred into the non-human sperm concurrently.

3. The method of Claim 1 wherein the exogenous DNA and type II restriction endonuclease are transferred into the non-human sperm sequentially.

4. The method of Claim 1 wherein said type II restriction endonuclease is *Not*I.

5. The method of Claim 1 wherein the exogenous DNA and type II restriction endonuclease are transferred into the non-human sperm by electroporation or lipofection.

6. The method of any of Claims 1-5 wherein the cohesive ends of said exogenous DNA have been formed by cleavage of circular DNA by a type II restriction endonuclease.

7. The method of any of Claims 1-5 wherein the cohesive ends of said exogenous DNA have been formed by cleavage of linear DNA by a type II restriction endonuclease.

8. The method of any of Claims 1-5 wherein the cohesive ends of said exogenous DNA have been formed by adding to each end of a linear DNA identical cohesive ends formed by chemical methods, identical cohesive ends formed by enzymatic methods, or cohesive ends formed by a combination of chemical and enzymatic methods.

9. The method of any of Claims 6-8 wherein one cohesive end is formed by cleavage by a type II restriction endonuclease, and wherein the other cohesive end of said exogenous DNA is formed by adding a cohesive end identical to the cohesive end formed by said type II restriction endonuclease cutting said exogenous DNA.

10. A method of producing non-human disease resistant animals comprising,
a. integrating an exogenous DNA into the sperm of an animal according to any of the methods of Claims 1-9, wherein said exogenous DNA confers disease resistance; and
b. using said non-human sperm to fertilize a non-human oocyte, and allowing said fertilized oocyte to develop into an animal.

11. The method of Claim 10, wherein the non-human oocyte is fertilized by means of *in vitro* fertilization.

12. The method of Claim 10, wherein the non-human oocyte is fertilized by means of artificial insemination.

13. The method of Claim 10 or 11, wherein said animal is mammalian or avian.

14. The method of Claim 13, wherein said mammal is bovine.

15. The method of Claim 13, wherein said avian is a chicken.

16. The method of any of Claims 10-15, wherein said exogenous DNA encodes a proteinaceous factor.

17. The method of Claim 16, wherein said proteinaceous factor comprises a single polypeptide or multiple polypeptides.

18. The method of any of Claims 10-15, wherein said exogenous DNA encodes a ribozyme.

19. The method of any of Claims 10-18, wherein said disease is caused by a viral pathogen.

20. The method of any of Claims 10-18, wherein said disease is caused by a bacterial pathogen.

21. The method of Claim 19, wherein said viral pathogen is the Infectious Bursal Disease Virus.

22. The method of Claim 21, wherein said exogenous DNA comprises the IBDV ORF Al sequence.

23. The method of claim 22, wherein said exogenous DNA comprises a plasmid encoding the IBDV ORF Al sequence under control of the citomegalovirus promoter, a neomycin resistance gene and an ampicillin resistance gene.

24. The method of Claim 16, wherein said protein is the IBDV ORF A1 protein.

25. The method of Claim 24, wherein said IBDV ORF A1 protein interferes with the production of pathogenic viral progeny

## Patentansprüche

1. Verfahren zum Integrieren exogener, doppelsträngiger DNA in genomische DNA eines Spermiums von nicht menschlicher Herkunft, umfassend:
a. Bilden kohäsiver Enden an der exogenen DNA, so dass besagte kohäsive Enden identisch mit den für eine DNA, die von einer bestimmten Typ-II-Restriktionsendonuklease geschnitten wurde, charakteristischen kohäsiven Enden sind, und
b. Transferieren besagter exogener DNA und besagter Typ-II-Restriktionsendonuklease in ein lebensfähiges Spermium.

2. Verfahren nach Anspruch 1, wobei die exogene DNA und die Typ-II-Restriktionsendonuklease gleichzeitig in das Spermium von nicht menschlicher Herkunft transferiert werden.

3. Verfahren nach Anspruch 1, wobei die exogene DNA und die Typ-II-Restriktionsendonuklease nacheinander in das Spermium von nicht menschlicher Herkunft transferiert werden.

4. Verfahren nach Anspruch 1, wobei es sich bei besagter Typ-II-Restriktionsendonuklease um *Not*I handelt.

5. Verfahren nach Anspruch 1, wobei die exogene DNA und die Typ-II-Restriktionsendonuklease mittels Elektroporation oder Lipofektion in das Spermium von nicht menschlicher Herkunft transferiert werden.

6. Verfahren nach einem der Ansprüche 1-5, wobei die kohäsiven Enden besagter exogener DNA durch Spaltung zirkulärer DNA mit einer Typ-II-Restriktionsendonuklease gebildet wurden.

7. Verfahren nach einem der Ansprüche 1-5, wobei die kohäsiven Enden besagter exogener DNA durch Spaltung linearer DNA mit einer Typ-II-Restriktionsendonuklease gebildet wurden.

8. Verfahren nach einem der Ansprüche 1-5, wobei die kohäsiven Enden besagter exogener DNA gebildet wurden, indem an jedes Ende einer linearen DNA identische kohäsive Enden, die mittels chemischer Verfahren gebildet wurden, identische kohäsive Enden, die mittels enzymatischer Verfahren gebildet wurden, oder kohäsive Enden, die mittels einer Kombination chemischer und enzymatischer Verfahren gebildet wurden, angefügt wurden.

9. Verfahren nach einem der Ansprüche 6-8, wobei ein kohäsives Ende durch Spaltung mit einer Typ-II-Restriktionsendonuklease gebildet wird und wobei das andere kohäsive Ende besagter exogener DNA gebildet wird, indem ein mit dem kohäsiven Ende, das durch das Schneiden besagter exogener DNA durch besagte Typ-II-Restriktionsendonuklease gebildet wird, identisches kohäsives Ende angefügt wird.

10. Verfahren zum Produzieren von krankheitsresistenten Tieren mit Ausnahme von Menschen, umfassend:
a. Integrieren einer exogenen DNA in das Spermium eines Tieres gemäß einem der Verfahren der Ansprüche 1-9, wobei besagte exogene DNA die Resistenz gegen die Krankheit überträgt, und
b. Verwenden besagten Spermiums von nicht menschlicher Herkunft zum Befruchten einer Oozyte von nicht menschlicher Herkunft und Ermöglichen, dass sich besagte befruchtete Oozyte zu einem Tier entwickelt.

11. Verfahren nach Anspruch 10, wobei die Oozyte von nicht menschlicher Herkunft mittels In-vitro-Fertilisation befruchtet wird.

12. Verfahren nach Anspruch 10, wobei die Oozyte von nicht menschlicher Herkunft mittels artifizieller insemination befruchtet wird.

13. Verfahren nach Anspruch 10 oder 11, wobei besagtes Tier ein Säuger oder Vogel ist.

14. Verfahren nach Anspruch 13, wobei besagter Säuger ein Rind ist.

15. Verfahren nach Anspruch 13, wobei besagter Vogel ein Huhn ist.

16. Verfahren nach einem der Ansprüche 10-15, wobei besagte exogene DNA für einen proteinartigen Faktor codiert.

17. Verfahren nach Anspruch 16, wobei besagter proteinartiger Faktor ein einziges Polypeptid oder mehrere Polypeptide umfasst.

18. Verfahren nach einem der Ansprüche 10-15, wobei besagte exogene DNA für ein Ribozym codiert.

19. Verfahren nach einem der Ansprüche 10-18, wobei besagte Erkrankung von einem viralen Krankheitserreger verursacht wird.

20. Verfahren nach einem der Ansprüche 10-18, wobei besagte Erkrankung von einem bakteriellen Krankheitserreger verursacht wird.

21. Verfahren nach Anspruch 19, wobei besagter viraler Krankheitserreger das Infektiöse Bursitis Virus ist.

22. Verfahren nach Anspruch 21, wobei besagte exogene DNA die Sequenz IBDV ORF A1 umfasst.

23. Verfahren nach Anspruch 22, wobei besagte exogene DNA ein Plasmid, das unter Kontrolle des Promotors des Cytomegalovirus für die Sequenz IBDV ORF A1 codiert, ein Gen für Resistenz gegen Neomycin und ein Gen für Resistenz gegen Ampicillin umfasst.

24. Verfahren nach Anspruch 16, wobei besagtes Protein das Protein des IBDV ORF A1 ist.

25. Verfahren nach Anspruch 24, wobei besagtes Protein des IBDV ORF A1 die Produktion der pathogenen viralen Abkömmlinge beeinträchtigt.

## Revendications

1. Procédé d'intégration d'ADN double brin exogène dans de l'ADN génomique de sperme non humain, comprenant:
a. la formation d'extrémités cohésives sur l'ADN exogène, de façon que lesdites extrémités cohésives soient identiques aux extrémités cohésives caractéristiques d'un ADN coupé par une endonucléase de restriction de type II donnée; et
b. le transfert dudit ADN exogène et de ladite endonucléase de restriction de type II dans du sperme viable.

2. Procédé selon la revendication 1, dans lequel l'ADN exogène et l'endonucléase de restriction de type II sont transférés simultanément dans le sperme non humain.

3. Procédé selon la revendication 1, dans lequel l'ADN exogène et l'endonucléase de restriction de type II sont transférés successivement dans le sperme non humain.

4. Procédé selon la revendication 1, dans lequel ladite endonucléase de restriction de type II est *Not*I.

5. Procédé selon la revendication 1, dans lequel l'ADN exogène et l'endonucléase de restriction de type II sont transférés dans le sperme non humain par électroporation ou lipofection.

6. Procédé selon l'une quelconque des revendications 1-5, dans lequel les extrémités cohésives dudit ADN exogène ont été formées par coupure d'ADN circulaire par une endonucléase de restriction de type II.

7. Procédé selon l'une quelconque des revendications 1-5, dans lequel les extrémités cohésives dudit ADN exogène ont été formées par coupure d'ADN linéaire par une endonucléase de restriction de type II.

8. Procédé selon l'une quelconque des revendications 1-5, dans lequel les extrémités cohésives dudit ADN ont été formées par addition, à chaque extrémité d'un ADN linéaire, d'extrémités cohésives identiques formées par des procédés chimiques, d'extrémités cohésives identiques formées par des procédés enzymatiques ou d'extrémités cohésives formées par une combinaison de procédés chimiques et enzymatiques.

9. Procédé selon l'une quelconque des revendications 6-8, dans lequel une extrémité cohésive est formée par coupure par une endonucléase de restriction de type II, et l'autre extrémité cohésive dudit ADN exogène est formée par addition d'une extrémité cohésive identique à l'extrémité cohésive formée par ladite endonucléase de restriction de type II coupant ledit ADN exogène.

10. Procédé de production d'animaux non humains résistants à une maladie, dans lequel:
a. on intègre un ADN exogène dans le sperme d'un animal selon l'un quelconque des procédés des revendications 1-9, ledit ADN exogène conférant une résistance à la maladie; et
b. on utilise ledit sperme non humain pour féconder un ovocyte non humain, et on laisse ledit ovocyte fécondé se développer dans un animal.

11. Procédé selon la revendication 10, dans lequel l'ovocyte non humain est fécondé par fécondation *in vitro.*

12. Procédé selon la revendication 10, dans lequel l'ovocyte non humain est fécondé par insémination artificielle.

13. Procédé selon la revendication 10 ou 11, dans lequel ledit animal est un mammifère ou un oiseau.

14. Procédé selon la revendication 13, dans lequel ledit mammifère est un bovin.

15. Procédé selon la revendication 13, dans lequel ledit oiseau est une poule.

16. Procédé selon l'une quelconque des revendications 10-15, dans lequel ledit ADN exogène code pour un facteur de type protéique.

17. Procédé selon la revendication 16, dans lequel ledit facteur de type protéique comprend un seul polypeptide ou des polypeptides multiples.

18. Procédé selon l'une quelconque des revendications 10-15, dans lequel ledit ADN exogène code pour un ribozyme.

19. Procédé selon l'une quelconque des revendications 10-18, dans lequel ladite maladie est provoquée par un pathogène viral.

20. Procédé selon l'une quelconque des revendications 10-18, dans lequel ladite maladie est provoquée par un pathogène bactérien.

21. Procédé selon la revendication 19, dans lequel ledit pathogène viral est le virus de la bursite infectieuse aviaire.

22. Procédé selon la revendication 21, dans lequel ledit ADN exogène comprend la séquence d'ORF A1 de l'IBDV.

23. Procédé selon la revendication 22, dans lequel ledit ADN exogène comprend un plasmide codant pour la séquence d'ORF A1 de l'IBDV sous le contrôle du promoteur du cytomégalovirus, un gène de résistance à la néomycine et un gène de résistance à l'ampicilline.

24. Procédé selon la revendication 16, dans lequel ladite protéine est la protéine ORF A1 de l'IBDV.

25. Procédé selon la revendication 24, dans lequel ladite protéine ORF A1 de l'IBDV perturbe la production de descendance virale pathogène.
